(19) Europäisches Patentamt
European Patent Office
Office européen des brevets

(11) Publication number : **0 418 697 B1**

# (12) EUROPEAN PATENT SPECIFICATION

(45) Date of publication of patent specification : **15.12.93 Bulletin 93/50**

(51) Int. Cl.$^5$ : **A61K 9/06,** A61K 47/18, A61K 37/30

(21) Application number : **90117394.8**

(22) Date of filing : **10.09.90**

## (54) Pharmaceutical compositions containing calcitonin.

The file contains technical information submitted after the application was filed and not included in this specification

(30) Priority : **15.09.89 IT 2172289**

(43) Date of publication of application : **27.03.91 Bulletin 91/13**

(45) Publication of the grant of the patent : **15.12.93 Bulletin 93/50**

(84) Designated Contracting States : **AT BE CH DE DK ES FR GB GR IT LI LU NL SE**

(56) References cited :
**DATABASE: CHEMICAL ABSTRACTS, accession no. 81-80457D, Derwent Publications Ltd, London, GB; & JP-A-56 118 013 (TEIJIN) 16-09-1981**
**DATABASE: CHEMICAL ABSTRACTS, accession no. 86-186015, Derwent Publicatons Ltd, London, GB; & JP-A-61 118 325 (YAMANOUCHI) 05-06-1986**

(73) Proprietor : **ESSETI s.a.s. LABORATORIO CHIMICO FARMACO BIOLOGICO DI A. IEVOLI & C.**
**Largo S. Maria La Nova, 19**
**I-80134 Napoli (IT)**

(72) Inventor : **Ventra, Ferdinando**
**Via Cavalli di Bronzo, 41**
**I-80046 S. Giorgio A Cremano (Napoli) (IT)**

(74) Representative : **Minoja, Fabrizio**
**Studio Consulenza Brevettuale Via Rossini, 8**
**I-20122 Milano (IT)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention concerns pharmaceutical compositions containing as active principle a calcitonin peptide.

Nowadays calcitonin are widely used for the treatment of Paget's disease, hypercalcemia and osteoporosis.

A disadvantageous feature of calcitonins, limiting the field of applications thereof, consists in their long-chain polypeptidic structure making them easily liable to degradation, in unsuited preservation or administration conditions. In addition, calcitonins must be preserved from the contamination by bacteria or other organisms or substances present in the environment as well as in the nasal mucosa, when the intranasal administration is used.

There is therefore the need of pharmaceutical compositions containing calcitonin which are stable and which may easily prevent the contamination by microorganisms and enzymes, responsible for the rapid degradation of calcitonin as well as the degradation by environmental agents both during the preparation of the formulation and during the administration which is generally carried out for prolonged periods and by means of a single multi-dose dispenser.

Many formulations for intra-nasal administration of calcitonin have been recently disclosed, characterized by the use of different kind of surfactants and/or absorption promoters (GB-A-2127689, EP-A-327756, EP-A-277462, EP-A-183527, EP-A-115627).

The known formulations are not however satisfactory in at least one of the following aspects: bioavailability, stability, tolerability, safety.

It has now been found that all the above requisites can be met by intra-nasal compositions of calcitonins containing one or more long-chain derivative of N-substituted aminoacids selected from alkyl-betaines, alkylamidobetaines, carboxylated alkylimidazolidines, alkylamidopropylbetaines, alkylglycines, having the following formulas

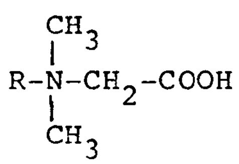

alkyl-betaine

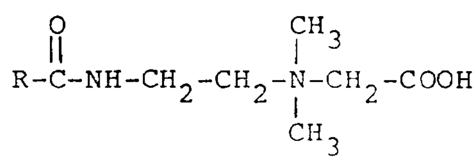

alkyl-amido-betaine

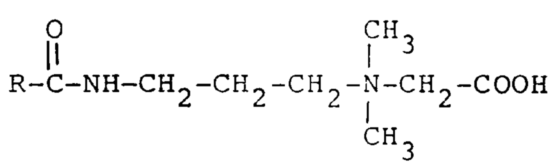

alkylamidopropyl betaine

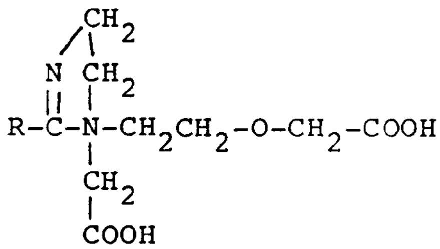

carboxylated alkylimidazolydine

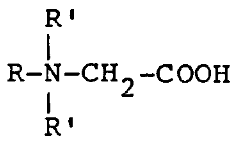

alkylglycine

wherein R and R' are alkyl residues, preferably $C_{10}$-$C_{24}$ alkyl residues.

These derivatives are widely used in the pharmaceutical industry as anti-septic and detergents for external use: their use has also been proposed for rectal compositions containing calcitonin (JP-A-56118013). The technical problems connected with the rectal administration are however completely different and the favourable effects obtained in the intranasal administration are therefore surprising.

The invention refers therefore to stable pharmaceutical compositions for nasal administrations containing:

a calcitonin,

a long-chain N-substituted amino-acid derivatives and optionally preserving agents (methyl or propyl p-oxybenzoates), and

a diluent or carrier suited for the application to the nasal mucosa.

According to the invention, the term "calcitonin" comprises natural or synthetic calcitonins or similar pharmaceutically active polypeptides. Salmon, eel, porcine and human calcitonin and elcatonin, particularly preferred, each of them being commercially available and described as far as their pharmacological activity is concerned.

The amount of calcitonin in the compositions of the invention depends on the kind of selected calcitonin, on the seriousness of the diseases to be treated and on the desired administration frequency.

The long-chain N-substituted amino-acid derivative may be contained in the amount from 2 to 10 mg per ml of solution.

A preferred class of N-substituted amino-acid derivative is that of alkylamidopropylbetaines.

The methyl-p-oxybenzoate may be contained in the amount from 1.2 to 1.3 mg per ml of solution.

The propyl-p-oxybenzoate may be contained in the amount from 0.3 to 0.2 mg per ml of solution.

The liquid diluent or carrier consist of an isotonic aqueous solution buffered at pH of about 4.0.

The compositions of the invention proved to be stable towards the microbial contamination from microorganisms such as E. coli, Staph. aureus and from yeasts such as Candida albicans and Aspergillus niger.

In the stability tests, the active principle proved to be stable after 2 years at a temperature of 27°C, in nitrogen atmosphere, in glass containers.

In the stability tests in comparison with 5 units of pancreatine per ml of solution, the compositions according to the invention were stable for an observation period of 30 days at room temperature, whereas the reference composition kept during the same period in the same conditions lost about 60% of biological activity.

In the clinical test the compositions of the invention did not induce side-effects.

In the bioavailability tests carried out administering to Rhesus monkeys 50 units of calcitonin, it was shown that the compositions of the invention exhibited plasma haematic levels about 35% higher than that of a reference composition, without long-chain N-substituted amino-acids.

The invention is further illustrated by the following Examples.

**EXAMPLE 1**

| | | |
|---|---|---|
| Synthetic salmon calcitonin | | 1400 UI |
| Sodium chloride | | mg 6.0 |
| Sodium citrate $2H_2O$ | | mg 4.63 |
| Citric acid $H_2O$ | | mg 4.54 |
| Methyl p-hydroxybenzoate | | mg 1.3 |
| Propyl p-hydroxybenzoate | | mg 0.2 |
| Sodium edetate | | mg 0.1 |
| Alkylbetaine | | mg 5.0 |
| Distilled water | to | mg 1.0 ml |

The methyl-p-hydroxybenzoate and propyl-p-hydroxybenzoate are dissolved in warm water. At about 20°C, under stirring, buffers, sodium edetate, sodium chloride, alkylbetaine and calcitonin are added to the solution in the order. The pH is adjusted to about 4.0 ± 0.3. The solution is filtered on Amberlite and then distributed in vials in a suited glass container of type 1, provided with a spray dispenser able to administer about 50UI for each application.

**EXAMPLE 2**

| | | |
|---|---|---|
| Synthetic salmon calcitonin | | 700 UI |
| Sodium chloride | | mg 6.0 |
| Sodium citrate $2H_2O$ | | mg 4.63 |
| Citric acid $H_2O$ | | mg 4.54 |
| Methyl p-hydroxybenzoate | | mg 1.2 |
| Propyl p-hydroxybenzoate | | mg 0.3 |
| Sodium edetate | | mg 0.1 |
| Alkyl amino betaine | | mg 7.5 |
| Distilled water | to | mg 1 ml |

The preparation process is the same as in Example 1.

## EXAMPLE 3

| | | |
|---|---|---|
| Synthetic salmon calcitonin | | 1400 UI |
| Sodium chloride | | mg 6.0 |
| Sodium citrate $2H_2O$ | | mg 4.60 |
| Citric acid $H_2O$ | | mg 4.54 |
| Methyl p-hydroxybenzoate | | mg 1.3 |
| Propyl p-hydroxybenzoate | | mg 0.2 |
| Sodium edetate | | mg 0.3 |
| Carboxylated alkylimidazolidine | | mg 2.5 |
| Alkylglycine | | mg 3.5 |
| Distilled water | to | mg 1 ml |

The preparation process is the same as in Example 1.

## EXAMPLE 4

| | | |
|---|---|---|
| Synthetic salmon calcitonin | | 1400 UI |
| Sodium chloride | | mg 6.0 |
| Sodium citrate $2H_2O$ | | mg 4.60 |
| Citric acid $H_2O$ | | mg 4.54 |
| Methyl p-hydroxybenzoate | | mg 1.3 |
| Propyl p-hydroxybenzoate | | mg 0.2 |
| Sodium edetate | | mg 0.3 |
| Alkylamidopropyl betaine | | mg 0.2 |
| Alkylglycine | | mg 3.5 |
| Distilled water | to | mg 1 ml |

## Claims

1. Pharmaceutical compositions for the intranasal administration of natural of synthetic calcitonins characterized by containing a long-chain N-subsituted amino acid.

2. Pharmaceutical compositions according to claim 1 wherein the N-substituted amino acid derivative is selected from alkyl-betaines, alkylamidobetaines, carboxylated alkylimidazolidines, alkylamidopropylbetaines, alkylglycines of formulae:

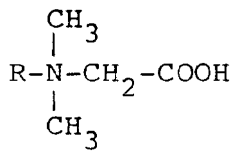

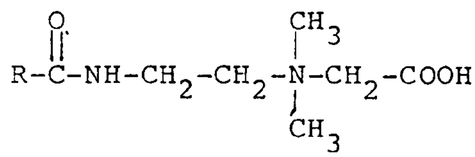

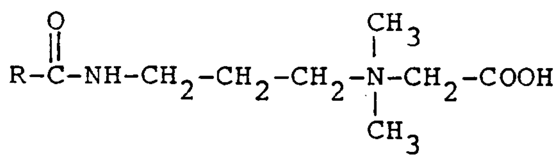

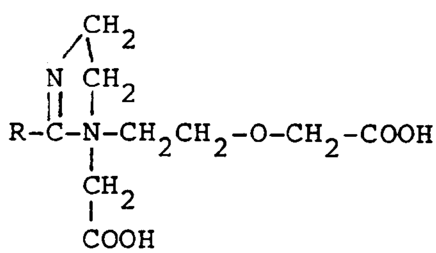

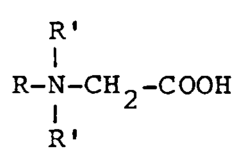

wherein R and R' are $C_{10}$-$C_{24}$ alkyl residues.

3. Pharmaceutical compositions according to claim 1 or 2, wherein the N-substituted amino acid is present in concentrations from 2 to 10 mg per ml of solution.

4. Pharmaceutical compositions according to any one of the previous claims, wherein calcitonin is selected from salmon, eel, porcine, human calcitonin and elcatonin.

5. Pharmaceutical compositions according to any one of the previous claims wherein the liquid diluent or carrier consists of an isotonic aqueous solution buffered at pH 4.0 ± 0.3.

6. Pharmaceutical compositions according to any one of the previous claims characterized by containing methyl-p-hydroxybenzoates or propyl-p-hydroxybenzoates.

7. Pharmaceutical compositions according to any one of the previous claims, characterized by containing calcitonin in amount from 50 to 400 UI.

## Patentansprüche

1. Pharmazeutische Zusammensetzungen zur intranasalen Verabreichung von natürlichen oder synthetischen Calcitoninen, **dadurch gekennzeichnet,** daß sie eine langkettige N-substituierte Aminosäure aufweist.

2. Pharmazeutische Zusammensetzungen nach Anspruch 1, wobei das N-substituierte Aminosäuren-Derivat aus Alkylbetainen, Alkylamidobetainen, karboxylierten Alkylimidazolinen, Alkylamidpropylbetainen, Alkylglycinen der folgenden Formeln ausgewählt wird:

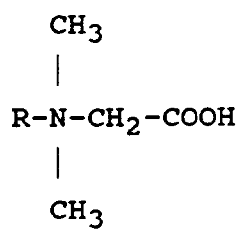

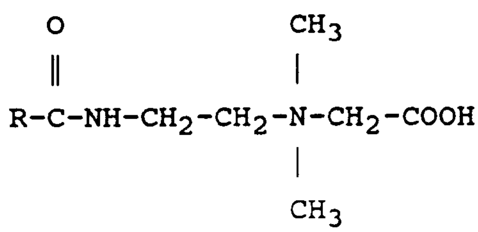

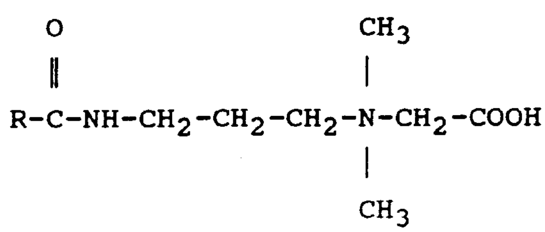

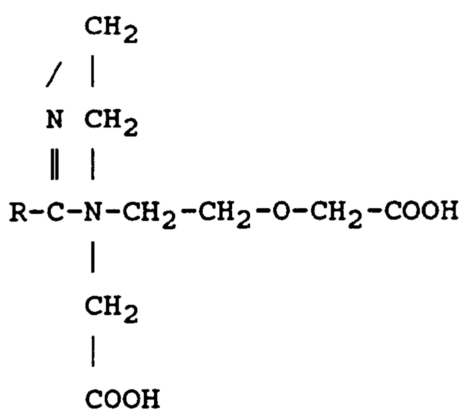

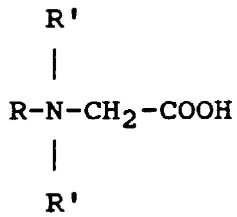

wobei R und R' $C_{10}$-$C_{24}$ Alkyl-Residuen sind.

3. Pharmazeutische Zusammensetzungen nach Anspruch 1 oder 2, wobei die N-substituierte Aminosäue in Konzentrationen von 2 bis 10 mg pro ml Lösung vorliegt.

4. Pharmazeutische Zusammensetzungen nach einem der vorhergehenden Ansprüche, wobei Calcitonin aus Calcitonin von Lachs, Aal, Schwein, Mensch und Elcatonin ausgewählt wird.

5. Pharmazeutische Zusammensetzungen nach einem der vorhergehenden Ansprüche, wobei der flüssige Lösungsvermittler oder Trägerstoff aus einer isotonischen wäßrigen Lösung besteht, die bei einem pH-Wert von $4{,}0 \pm 0{,}3$ gepuffert ist.

6. Pharmazeutische Zusammensetzungen nach einem der vorhergehenden Ansprüche, **gekennzeichnet durch** das Beinhalten von Methyl-p-Hydroxybenzoaten oder Propyl-p-Hydroxybenzoaten.

7. Pharmazeutische Zusammensetzungen nach einem der vorhergehenden Ansprüche, **gekennzeichnet durch** das Beinhalten von Calcitonin in einer Menge von 50 bis 400 I.E.

## Revendications

1. Compositions pharmaceutiques pour l'administration intranasale de calcitonines naturelles ou synthétiques, caractérisées en ce qu'elles contiennent un amino-acide N-substitué à longue chaîne.

2. Compositions pharmaceutiques selon la revendication 1, dans lesquelles le dérivé d'amino-acide N-substitué est choisi parmi les alkyl-bétaïnes, alkylamidobétaïnes, alkylimidazolidines, carboxylées, alkylamidopropylbétaïnes, alkylglycines de formule :

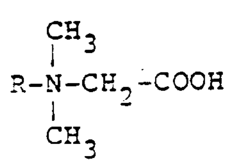

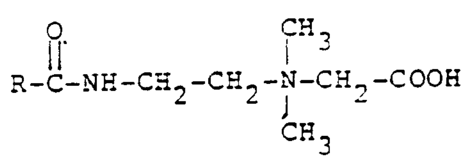

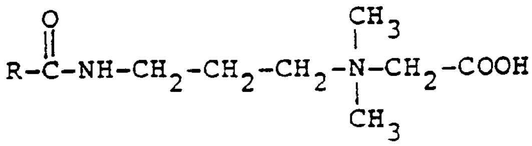

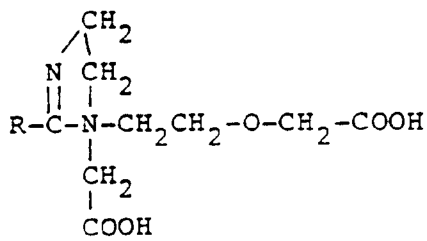

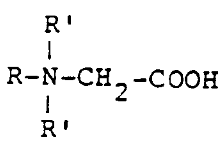

où R et R' sont des radicaux alkyles en $C_{10}$ - $C_{24}$.

3. Compositions pharmaceutiques selon la revendication 1 ou 2, dans lesquelles l'amino-acide N-substitué est présent en des concentrations de 2 à 10 mg par ml de solution.

4. Compositions pharmaceutiques selon l'une quelconque des revendications précédentes, dans lesquelles la calcitonine est choisie parmi la calcitonine et l'elcatonine de saumon, d'anguille, de porc, et humaines.

5. Compositions pharmaceutiques selon l'une quelconque des revendications précédentes, dans lesquelles le diluant ou véhicule liquide consiste en une solution aqueuse isotonique tamponnée à pH 4, O ± 0,3.

6. Compositions pharmaceutiques selon l'une quelconque des revendications précédentes, caractérisées en ce qu'elles contiennent des p-hydroxybenzoates de méthyle ou des p-hydroxybenzoates de propyle.

7. Compositions pharmaceutiques selon l'une quelconque des revendications précédentes, caractérisées en ce qu'elles contiennent de la calcitonine à raison de 50 à 400 UI.